# EUROPÄISCHE PATENTSCHRIFT

(11) **EP 1 168 957 B1**
(45) Veröffentlichungstag und Bekanntmachung des Hinweises auf die Patenterteilung: **09.11.2005**
(21) Anmeldenummer: 00941973.0
(22) Anmeldetag: 22.05.2000
(51) Int. Cl.: A61B 1/00, G02B 23/24

(54) **ENDOSKOP MIT VERSTELLBAREM BAUELEMENT**
ENDOSCOPE WITH A MOVABLE COMPONENT
ENDOSCOPE A COMPOSANT MOBILE

(30) Priorität: 18.06.1999 DE 19927816
(43) Veröffentlichungstag der Anmeldung: 09.01.2002
(73) Patentinhaber: OLYMPUS WINTER & IBE GMBH, 22045 Hamburg (DE)
(72) Erfinder: WULFSBERG, Jens, Peter, D-23843 Neritz (DE)
(74) Vertreter: Schaefer, Konrad
(86) Internationale Anmeldenummer: PCT/EP2000/004634
(87) Internationale Veröffentlichungsnummer: WO 2000/078205

(56) Entgegenhaltungen:
- DE-A- 19 521 654
- DE-A- 19 618 355
- DE-A- 19 713 276
- DE-U- 8 810 044
- US-A- 5 836 867

## Beschreibung

Die Erfindung betrifft ein Endoskop der im Oberbegriff des Anspruchs 1 genannten Art.

Gattungsgemäße Endoskope weisen innerhalb einer abgedichteten Gehäusewand Einrichtungen auf, die von Umgebungseinflüssen zu schützen sind. Solche Einrichtungen sind insbesondere bildgebende Einrichtungen, wie Linsenoptiken oder elektronische Kameras. Lichtübertragungseinrichtungen, wie z.B. Lichtleitfaserbündel oder sonstige empfindliche optische oder elektronische Einrichtungen. Diese Einrichtungen sind in dem von der abgedichteten Gehäusewand umschlossenen Raum vor Feuchtigkeit und Dampf aus der Umgebung geschützt.

Solche gattungsgemäßen Endoskope werden insbesondere in der Medizin eingesetzt, wo das Gehäuse Schutz vor Körperflüssigkeiten und bei der Reinigung und Sterilisation des Endoskopes bietet.

Die Verstellung des Bauelementes mittels magnetischem Durchgriff durch die unmagnetische Gehäusewand ermöglicht eine Verstellung des Bauelementes von außen, ohne daß dazu Schiebedurchführungen oder sonstige hinsichtlich der Gehäuseabdichtung problematische Einrichtungen erforderlich sind. Es ergibt sich eine sehr gut abdichtbare Gehäusekonstruktion, die insbesondere auch sehr häufige Heißdampfautoklavierung zur vollständigen Sterilisation ermöglicht.

Mit dem verstellbarem Bauelement lassen sich im Inneren des Endoskopes elektrische Schalter betätigen, Ventile für z.B. Saug- oder Spülleitungen schalten und insbesondere optische Einrichtungen verstellen, beispielsweise zur Fokussierung.

Gattungsgemäße Endoskope sind aus der US-A-5 836867, der DE 8810044U, der DE 197 13 276 A1 und der DE 195 21 654 A1 bekannt. Der Triebkörper ist bei diesen Konstruktionen ebenfalls als Permanentmagnet ausgebildet. Im Falle der drittgenannten bekannten Konstruktion sind mehrere Paare innerer und äußerer Magneten vorgesehen, die eine winkelgenaue Kopplung ergeben sollen. Der innen magnetisch gedrehte Ring greift in ein Verstellgewinde ein und ist dadurch reibgesichert. Bei der zweitgenannten bekannten Konstruktion gleitet ein innerer Magnet in einer Kurvenbahn, die ebenfalls eine Reibsicherung ergeben soll. Hierdurch soll verhindert werden, daß sich das Bauelement bei Stößen verstellt, die die inneren und äußeren Magnete außer Eingriff bringen. Exakte Einstellpositionen des Bauelementes lassen sich mit diesen Konstruktionen nicht sichern, da der innenliegende Magnet in jeder beliebigen Position stehenbleiben kann.

Unabdingbar ist bei den bekannten Konstruktionen die dauernde Lagerung des Magneten am Endoskop. Die dazu erforderliche, am Endoskop vorgesehene Stelleinrichtung, die den Magneten enthält, ergibt jedoch zwischen Stelleinrichtung und Endoskop schwer zugängliche Zwischenräume und Spalten, die schwierig zu reinigen und zu sterilisieren sind.

Da die Stelleinrichtung ständig am Endoskop verbleiben muß, auch wenn sie nicht zur Verstellung benötigt wird, kann sie die Handhabung des Endoskopes stören und belastet das Endoskop.

Außerdem ist eine solche Stelleinrichtung nur am Hauptkörper vorsehbar und kann lediglich Bauelemente im Hauptkörper verstellen. Im Schaftbereich des Endoskopes, also insbesondere an dem im distalen Endbereich des Schaftes vorgesehenen Objektiv, kann auf die bekannte Weise nicht verstellend eingegriffen werden, da dort außenliegende Stelleinrichtungen, die dauernd am Schaft verbleiben müssen, nicht möglich sind.

Aus der DE 196 18 355 A ist ein nicht gattungsgemäßes Endoskop bekannt, bei dem in das Gehäuse ein das Objektiv umgebener Permanentmagnet eingebaut ist, der das Objektiv über einen an diesem angeordneten Triebkörper bei Strombeaufschlagung verstellt. Hierzu sind jedoch nach außen geführte, schwierig abzudichtende elektrische Anschlüsse erforderlich.

Die Aufgabe der vorliegenden Erfindung besteht darin, ein gattungsgemäßes Endoskop zu schaffen, das nicht durch äußere Stelleinrichtungen belastet ist, keine elektrischen Anschlüsse benötigt und zur Verstellung im distalen Endbereich des Schaftes geeignet ist.

Diese Aufgabe wird mit den Merkmalen des Anspruches 1 gelöst.

Erfindungsgemäß ist das Bauelement in bestimmten, konstruktiv vorgegebenen Stellpositionen mechanisch gesichert und läßt sich mit Magnetkraft unter Überwindung der Haltekraft der Sicherungseinrichtung magnetisch verstellen. Der von außen einwirkende Magnet ist gesondert vom Endoskop vorgesehen und muß nur zur Verstellung in Magneteingriff gebracht werden. Anschließend kann er wieder entfernt werden, ohne Gefährdung der neu eingestellten Stellposition des Bauelementes, die von der Sicherungseinrichtung gehalten wird. Das Endoskop kann daher unbelastet von einer äußeren Stelleinrichtung verwendet werden. Insbesondere ist es damit auch möglich, ein zu verstellendes Bauelement im Schaftbereich des Endoskopes, und zwar insbesondere im Bereich des Objektives, vorzusehen, wozu bisher keinerlei konstruktive Möglichkeiten bekannt waren.

Die Sicherungseinrichtung kann beispielsweise als Reibbremse, die die Stellung des Bauelementes sichert, ausgebildet sein, ergänzt um einen die exakte Einstellung der Position ermöglichenden Endanschlag. Es sind auch magnetische Sicherungseinrichtungen möglich, die bestimmte Positionen festhalten. Vorzugsweise sind jedoch die Merkmale des Anspruches 2 vorgesehen. Federrasteinrichtungen lassen sich konstruktiv sehr einfach zur Sicherung bestimmter Stellungen des Bauelementes vorsehen.

Erfindungsgemäß läßt sich insbesondere auch im distalen Schaftbereich am Objektiv verstellend eingreifen. Dort können erforderliche Verstellungen am Objektiv vorgenommen werden, wie beispielsweise eine Fokusverstellung durch axiale Längsverstellung von Teilen des Linsensystems. Vorzugsweise sind jedoch die Merkmale des Anspruches 3 vorgesehen, womit sich eine Verstellung der Blickrichtung des Endoskopes bewirken läßt. Damit läßt sich ein lange gehegter Wunsch der Chirurgen erfüllen, nämlich auf einfache Weise an einem Endoskop die Blickrichtung zu verstellen, beispielsweise von geradeausblickend in schrägblickend. Es muß dazu das Endoskop nicht gewechselt werden, sondern nur bei kurzer Unterbrechung der Operation mit einem Magneten in der Nähe der distalen Spitze des Endoskopes eine Stellbewegung durchgeführt werden.

In den Zeichnungen ist die Erfindung beispielsweise und schematisch dargestellt Es zeigen:
- Fig 1:: einen Achsschnitt durch den distalen Endbereich eines Endoskopes mit außen angeordnetem Magneten zur Fokusverstellung und
- Fig. 2: einen Schnitt gemäß Figur 1 durch ein Endoskop mit Magnet zur Blickrichtungsverstellung.

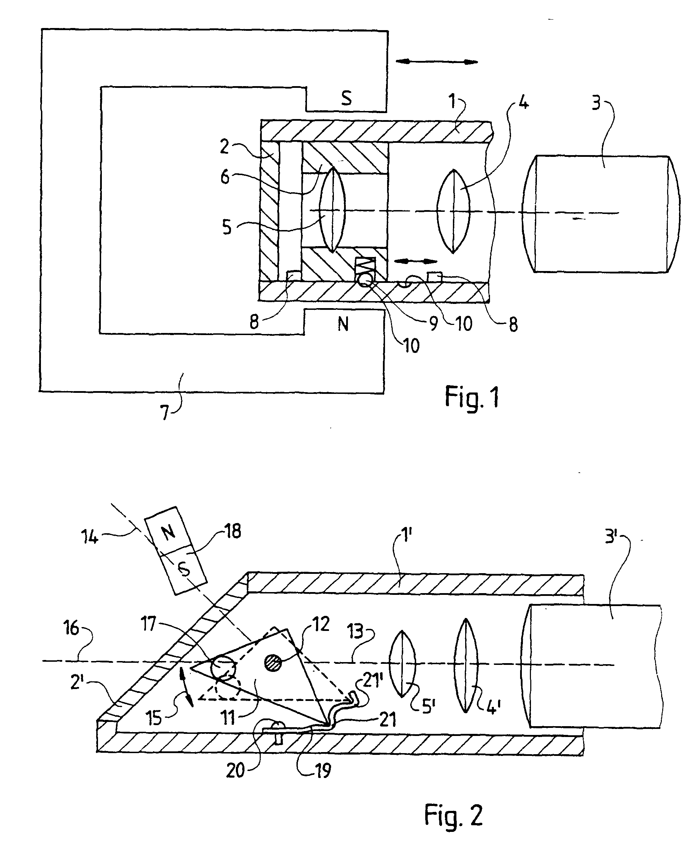

Figur 1 zeigt den distalen Endbereich des Schaftteiles eines medizinischen Endoskopes mit einem den Schaft ausbildenden Rohr 1, das an seinem distalen Ende mit einem Fenster 2 abgedichtet verschlossen ist. Im Inneren des Rohres 1 ist als Bildleiter im dargestellten Ausführungsbeispiel eine Stablinsenanordnung vorgeselten, von der eine Stablinse 3 dargestellt ist. Es ist ein Objektiv vorgesehen, das im Ausführungsbeispiel aus zwei Objektivlinsen 4 und 5 besteht. Die Stablinse 3 und die Objektivlinse 4 sind mit nicht dargestellten Mitteln im Rohr 1 befestigt.

Die distale Objektivlinse 5 ist in Achsrichtung des Rohres 1 verschiebbar in diesem gelagert Sie ist dazu in einem Gleitring 6 gefaßt, welcher, wie dargestellt, im Rohr 1 axial verschiebbar geführt ist.

Das Rohr 1 besteht aus für Magnetfelder durchlässigem, also unmagnetischem Material, wie beispielsweise geeignet legiertem Edelstahl, der auch als Schaftmaterial für Endoskope geeignet ist. Der Gleitring 6 ist als von einem Magnetfeld bewegbarer Triebkörper ausgebildet und besteht beispielsweise aus einem magnetisch hochpermeablem Material, wie z.B. für Transformatorenbleche verwendetem Eisen, auf das Magnetfelder hohe Kräfte ausüben.

Es ist eine magnetische Stelleinrichtung vorgesehen in Form eines vom Endoskop konstruktiv getrennten Magneten 7, der im Ausführungsbeispiel als mit einem U-förmigen Joch geschlossener Permanentmagnet mit Polen S und N ausgebildet ist. *Z*wischen den Polen S und N durchläuft ein Magnetfeld einen Luftspalt, der beim dargestellten Magneten 7 die Breite des Außendurchmessers des Rohres 1 aufweist.

Wird der Magnet 7 in die dargestellte Lage mit dem distalen Endbereich des Rohres 1 innerhalb des Magnetspaltes gebracht, so durchfließt das vom Magneten produzierte Magnetfeld den Gleitring 6 und übt auf diesen starke Kräfte aus.

Wird der Magnet 7 in Achsrichtung des Rohres 1 bewegt, so wird der Gleitring 6 durch magnetische Kräfte mitbewegt. Die distale Objektivlinse 5 kann also gegenüber der feststehenden Objektivlinse 4 verstellt werden, wodurch je nach Ausbildung des Objektivs und je nachdem, ob die eine oder andere der beiden Linsen des Objektivs auf die dargestellte Weise verschiebbar ausgebildet ist, eine Fokuseinstellung oder dergleichen erreichbar ist.

Nach gewünschter axialer Verstellung der verschiebbaren Objektivlinse 5 kann der Magnet 7 entfernt werden. Der Gleitring 6 wird dann nicht mehr magnetisch beeinflußt und ist somit kraftfrei. Er bleibt dann in der neu eingestellten Position stehen.

Wäre der Gleitring 6 im Inneren des Rohres 1 ungesichert frei verschiebbar, so könnte er unter Einwirkung von Beschleunigungskräften, beispielsweise bei kräftigen Bewegungen des Endoskopes oder beispielsweise beim Anschlagen des Endoskopes gegen eine Tischkante sich unter Wirkung der Beschleunigungskräfte von selbst verstellen. Außerdem ist bei einer Bewegung des Magneten 7 außerhalb des Rohres 1 nicht von außen ersehbar, in welcher Position der Gleitring 6 im Inneren des optisch undurchsichtigen Rohres 1 steht. Beim Verstellen wüßte man nie genau, wo der Gleitring 6. der vom Magnetfeld relativ unpräzise mitgenommen wird, steht

Daher sind im Ausführungsbeispiel im Inneren des Rohres 1 zwei Endanschläge 8 befestigt, gegen die der Gleitring 6 in Endanlage seiner axialen Bewegung gelangen kann. Bewegt man den Magneten 7 nur weit genug in die eine oder andere Richtung, dann besteht Sicherheit, daß der Ring 6 wirklich bis in Anschlag an einen der Endanschläge 8 bewegt ist.

An den Endanschlägen 8 muß der Gleitring 6 aber immer noch gegen Belegung durch Beschleunigungskräfte gesichert werden. Dies kann beispielsweise durch eine Reibbremse erfolgen. Der Gleitring 6 kann mit ausreichender Reibkraft im Inneren des Rohres 1 gelagert sein, so daß er vom Magnetfeld des Magneten 7 noch bewegbar ist, bei Beschleunigungskräften aber durch die Reibkraft gehalten wird.

Im Ausführungsbeispiel ist zur Sicherung der beiden Endpositionen des Gleitringes 6, zwischen denen er verstellbar sein soll, eine Federrasteinrichtung vorgesehen. Diese weist im Gleitring 6 in einem axialen Kanal eine von einer dargestellten Feder radial nach außen gedrückte Kugel 9 auf, sowie zwei Rastvertiefungen 10 in der Innenfläche des Rohres 1, in die die federabgestützte Kugel 9 in den axialen Endpositionen des Gleitringes 6 eimasten kann. Mit der die Kugel 9 beaufschlagenden Feder kann die Ausrastkraft präzise definiert werden, so daß sichergestellt ist, daß bis zu bestimmten Beschleunigungskräften der Gleitring 6 gehalten wird, mit dem Magnetfeld des Magneten 7 jedoch unter Überwindung der Rastkraft eine Verstellung aus der einen in die andere axiale Position möglich ist. Die Endschlage 8 können bei dieser Konstruktion auch entfallen.

Die in Figur 1 dargestellte Konstruktion ermöglicht, das Objektiv 4, 5 zwischen zwei Einstenungen präzise hin- und herzuschalten.

Figur 2 zeigt in einer zweiten Ausführungsform der Erfindung den distalen Endberoch des Schaftteiles eines Endoskopes mit einem Rohr 1', das den Schaft ausbildet und in dem mit nicht dargestellten Mitteln eine Stablinse 3' und zwei Objektivlinsen 4', 5' befestigt sind. Vor dem Objektiv 4', 5' ist als verstellbares Bauelement ein Umlenkprisma 11 angeordnet, das mit einer senkrecht zur Zeichnungsebene stehenden Welle 12 am Rohr 1' drehbar gelagert ist. Ein Fenster 2' verschließt schräg zur Achse des Rohres 1' stehend dessen distale Öffnung. Steht das Prisma 11 in der mit ausgezogenen Linien dargestellten Drehlage, so lenkt es die optische Achse 13 des Systems in eine schräge Blickrichtung durch das Fenster 2' in Richtung der gestrichelten Linie 14. Wird es, wie gestricheit dargestellt, entgegen dem Uhrzeigersinn um den mit dem Pfeil 15 angegebenen Winkel gedreht, so lenkt es die optische Achse 13 in die neue geradeausblickende Blickrichtung 16. Durch Verdrehen des Prismas 1 kann also die Blickrichtung des Endoskopes von schrägbhckend zu geradeausblickend um einen Winkel verstellt werden. Das Prisma 11 ist in diesem Fall als Dove-Prisma ausgeführt. Es kann auch eine andere durch Winkelverstellung die Blickrichtung umschaltende Einrichtung verwendet werden, die z.B. mit Drehspiegeln oder Klappspiegeln arbeitet und entsprechend zur Blickrichtungsverstellung gedreht oder geschwenkt wird.

Am Prisma 11 ist außerhalb der Welle 12, also außerhalb seiner Drehachse, ein von einem Magnetfeld bewegbarer Triebkörper 17 befestigt, der beispielsweise aus demselben Material besteht, das bei der Ausführungsform der Figur 1 für den Gleitring 6 angegeben ist. Als konstruktiv getrennte äußere Stelleinrichtung ist ein einfacher Permanentmagnet 18 dargestellt, der mit seinem Magnetfeld durch das Fenster 2' bzw. durch die, wie zum Ausführungsbeispiel der Figur 1 beschrieben, unmagnetische Wand des Rohres 1' greift.

Durch Annäherung des Magneten 18 bzw. durch Bewegen des Magneten 18 in einer kreisförmigen Bahn um die in Figur 2 dargestellte Spitze des Endoskopes herum, kann der Triebkörper 17 bewegt und das Prisma 11 gedreht werden.

Aus den bei der Beschreibung der Figur 1 genannten Gründen ist es schwierig, von außen die beiden gewollten Winkelpositionen des Prismas 11 genau einzustellen, in denen die gewünschte Blickrichtung in Richtung der gestrichelten Linien 14 bzw. 16 steht Außerdem muß das Prisma 11 in den eingestellten Positionen gegen ungewollten Verstellung durch Beschleunigungskräfte gesichert sein.

Dazu ist im Ausführungsbeispiel eine Federrasteinrichtung vorgesehen mit einer Blattfeder 19, die an einem Ende mit dem dargestellten Niet 20 an der Wand des Rohres 1' befestigt ist. An ihrem freien Ende ist die Blattfeder 19 wellenförmig mit zwei Rastmulden 21, 21' geformt, in die die dem Triebkörper 17 benachbarte Ecke des Prismas 11 federnd einrasten kann. Bei der ausgezogen dargestellten Winkelposition des Prismas 11 ist diese Ecke in der Rastmulde 21 eingerastet. Nach magnetischer Drehverstellung des Prismas 11 um den Winkel 15 rastet die Ecke in die andere Rastmulde 21' ein, wie in der Figur dargestellt. Nach Rastverstellung kann der Magnet 18 entfernt werden und die neue Winkelposition des Prismas 11 wird von der Rasteinrichtung bis zur nächsten Verstellung gesichert.

Die Triebkörper 6, 17 der beiden dargestellten Ausführungsformen können auch als langgestreckte Körper aus magnetisch permeablem Material ausgebildet sein, die sich aufgrund ihrer Formanisotropie stets in Richtung der Feldlinien eines einwirkenden äußeren Magnetfeldes ausrichten und durch Drehung des einwirkenden Magnetfeldes drehverstellt werden. Diese Ausführungsform läßt sich insbesondere bei der Konstruktion der Figur 2 gut verwenden. Bei der Ausführungsform der Figur 1 könnte der Gleitring 6 aus unmagnetischem Material bestehen und einen quer zur Achse des Rohres 1 erstreckten Triebkörper enthalten, mit dem der Ring 6 gedreht werden kann. Mit einer solchen Konstruktion könnte beispielsweise ein Polarisationsfilter gedreht werden.

Die Triebkörper 6, 17 der dargestellten Ausführungsformen können auch als Permanentmagnete ausgebildet sein, welche mit sehr hohen Kräften auf einwirkende äußere Magnetfelder reagieren.

In den dargestellten Ausführungsformen sind innere verstellbare Bauelemente 5, 11 im distalen Endbereich des Schaftteiles eines Endoskopes dargestellt. Ähnliche Konstruktionen können jedoch auch im proximalen, an den Schaftbereich anschließenden Hauptkörper eines Endoskopes vorgesehen sein, um don z.B. Okularlinsen zu verstellen oder beispielsweise ein Umlenkprisma zu drehen, mit dem der Strahiengang zwischen zwei Betrachtungseinrichtungen verschwenkbar ist, beispielsweise einer Kamera und einem Okular. Es können mit solchen magnetischen Verstelleinrichtungen auch andere erforderliche Betätigungen im Inneren des Endoskopes vorgenommen werden, beispielsweise können elektrische Schaher verstellt werden oder Ventile an den Innenraum des Endoskopes durch laufende Gas- oder Flussigkeitsleitungen geschaltet werden.

In den dargestellten Ausführungsbeispielen sind an den zu verstellenden Bauelementen 5, 11 je em magnetisch bewegbarer Triebkörper 6, 17 vorgesehen. Es können auch mehrere Triebkörper vorgesehen sein, die beispielsweise von außen auch von mehreren Magneten anziehend oder abstoßend bewegt werden können.

## Patentansprüche

1. Endoskop mit abgedichteter Gehäusewand (1, 1'), mit einem außerhalb der Gehäusewand (1, 1') angeordneten Magneten (7, 18) und mit einem innerhalb der Gehäusewand (1, 1') angeordneten Bauelement (5, 11), das mit einem am Bauelement befestigten, von einem Magnetfeld bewegbaren Triebkörper (6, 17) durch die Wand hindurch von dem Magneten (7, 18) verstellbar ist, **dadurch gekennzeichnet, daß** das Bauelement (5, 11) mit einer Sicherungseinrichtung (8, 9, 10; 19, 21, 21') in bestimmten Stellpositionen mit einer von dem Magneten (7, 18) überwindbaren Haltekraft mechanisch gesichert ist, und daß der Magnet (7, 18) als vom Endoskop (1, 1') konstruktiv getrenntes, in Magneteingriff bringbares Konstruktionsteil ausgebildet ist.

2. Endoskop nach Anspruch 1, **dadurch gekennzeichnet, daß** die Sicherungseinrichtung als Federrasteinrichtung (9, 19) ausgebildet ist.

3. Endoskop nach Anspruch 1, **dadurch gekennzeichnet, daß** das verstellbare Bauelement als im distalen Endbereich des Endoskopes (1') am Objektiv (4', 5') angeordnete Schwenkeinrichtung (11, 12) zur Verstellung der Blickrichtung (14, 16) des Endoskopes ausgebildet ist.

## Claims

1. Endoscope with a sealed housing wall (1, 1'), with a magnet (7, 18) disposed outside the housing wall (1, 1') and with a component (5, 11) which is disposed inside the housing wall (1, 1') and is adjustable through the wall by the magnet (7, 18) by means of a drive member (6, 17) which is fixed on the component and is movable by a magnetic field, **characterised in that** the component (5, 11) is mechanically secured in specific adjusted positions by means of a securing device (8, 9, 10; 19, 21, 21') with a retaining force which can be overcome by the magnet (7, 18), and that the magnet is constructed as a structural part which is structurally separate from the endoscope (1, 1') and can be brought into magnetic engagement.

2. Endoscope as claimed in Claim 1, **characterised in that** the securing device is constructed as a spring-loaded detent arrangement (9, 19).

3. Endoscope as claimed in Claim 1, **characterised in that** the adjustable component is constructed as a pivoting device (11, 12) disposed in the distal end zone of the endoscope (1') on the objective (4', 5') for the adjustment of the viewing direction (14, 16) of the endoscope.

## Revendications

1. Endoscope à enveloppe (1, 1') étanchéifiée doté d'un aimant (7, 18) agencé à l'extérieur de l'enveloppe (1, 1') et d'un composant (5, 11) agencé à l'intérieur de l'enveloppe (1, 1'), lequel est ajustable à travers l'enveloppe au moyen de l'aimant (7, 18) par l'intermédiaire d'un élément moteur (6, 17) fixé au composant et pouvant être actionné par un champ magnétique, **caractérisé en ce que** le composant (5, 11) est, au moyen d'un dispositif d'arrêt (8, 9, 10; 19, 21, 21'), mécaniquement bloqué dans des positions d'action définies où il est maintenu par une force de rétention pouvant être surmontée par l'aimant (7, 18), et **en ce que** l'aimant (7, 18) est réalisé sous forme d'élément de construction séparé de l'endoscope (1, 1') et pouvant être placé en position d'accouplement magnétique.

2. Endoscope selon la revendication 1, **caractérisé en ce que** le dispositif d'arrêt est réalisé sous forme de d'encliquetage à ressort (9, 19).

3. Endoscope selon la revendication 1, **caractérisé en ce que** le composant ajustable est réalisé sous forme de dispositif de pivotement (11, 12) agencé dans la section d'extrémité distale de l'endoscope (1') au niveau de l'objectif (4', 5') pour le réglage de la direction de visée (14, 16) de l'endoscope.
